# EUROPEAN PATENT APPLICATION

(11) **EP 1 519 189 A1**
(43) Date of publication of application: **30.03.2005**
(21) Application number: 03733576.7
(22) Date of filing: 26.06.2003
(51) Int. Cl.: G01N 23/225, G01N 33/53

(54) **METHOD OF ANALYZING PROBE SUPPORT BY USING FLYING TIME SECONDARY ION MASS SPECTROMETRY**

(30) Priority: 28.06.2002 JP 2002190010; 28.06.2002 JP 2002191391; 28.06.2002 JP 2002191414
(71) Applicant: CANON KABUSHIKI KAISHA, Ohta-ku Tokyo 146-8501 (JP)
(72) Inventor: OKAMOTO, Tadashi, Tokyo 146-8501 (JP); TAKASE, Hiromitsu, Tokyo 146-8501 (JP); HASHIMOTO, Hiroyuki, Tokyo 146-8501 (JP)
(74) Representative: Beresford, Keith Denis Lewis
(86) International application number: PCT/JP2003/008104
(87) International publication number: WO 2004/003532

(57) **Abstract**

A method of analyzing a measuring sample is provided which is capable of accurately analyzing the state of a probe disposed on a carrier and formation/unformation of a hybrid between the probe and a target nucleic acid, for example, imaging of the disposing locations and quantitative analysis thereof.

The state of a nucleic acid probe formed in a measuring sample obtained by reacting a sample with a probe carrier or formation/unformation of a hybrid between the probe and a target nucleic acid is detected by measurement by the Time-of-Flight Secondary Mass Spectroscopy while being labeled with a marker substance capable of generating fragment ions that are not generated by fragmentation of the probe or the target substance.

## Description

### BACKGROUND OF THE INVENTION

### Field of the Invention

The invention relates to a method of analyzing a probe carrier using the Time-of-Flight Secondary Ion Mass Spectrometry, particularly to a method of analyzing the state of probes immobilized in a matrix pattern on the probe carrier by the Time-of-Flight Secondary Ion Mass Spectrometry, for example a method for imaging a number of matrixes on the surface of the probe carrier on which nucleic acid probes are immobilized, or a method for quantitatively analyzing the nucleic acid probes constituting the matrixes. The invention also relates to a method for detecting and analyzing target nucleic acids using a so-called nucleic acid chip on which a plurality of nucleic acid probes are disposed on a substrate in a matrix pattern.

### Related Background Art

Nucleic acid chips such as DNA chips and RNA chips as examples of probe carriers have been used for genomic analysis or analyzing expression of genes. The analysis results are expected to provide important indices for diagnosis, prognosis and determination of therapeutic policy of cancers, hereditary diseases, life style diseases and infectious diseases.

Several methods are known for preparing the nucleic acid chip. For example, representative methods for preparing DNA chips include a successive synthesis method of the DNA probe on the substrate using photolithography (such as in U.S. Patent No. 5,405,783), and an immobilizing method of previously synthesized DNA or cDNA (complementary DNA) by feeding it on the substrate (such as in U.S. Patent No. 5,601,980, Japanese Patent Application Laid-Open No. H11-187900 and Science Vol. 270, 467, 1995).

Usually, the nucleic acid chip is prepared by any of the methods described above. A desired object can be attained by detecting formation/unformation of a hybrid of the nucleic probe and a target nucleic acid on the nucleic acid chip by some methods after the chip and a solution containing a nucleic acid to be detected, or a target nucleic acid, has been left in a hybridization condition.

It is crucial for assuring reliability, or a quantitative property and reproducibility, of the analysis, to determine the quantity of either the nucleic acid probe or the target nucleic acid hybridized with the nucleic acid probe, or both quantities existing in the matrix, or the density of the nucleic acid probe and hybridized target nucleic acid. It is also important from the viewpoint of ensuring the quantitative property and reliability to be informed of actual configuration (imaging) of the matrix form (shape, size and state).

Suppose that no physical address indicating the positions of the matrixes is formed on the substrate for forming the chip. Then, the analysis portion of the chip cannot be distinguished depending on the detection methods due to the absence of the physical address as will be particularly described hereinafter, when the chip is prepared by supplying a probe solution as fine droplets using, for example, an ink-jet method. For solving such problem, the position of the matrix should be visualized by the detection method itself employed.

However, the nucleic acid probe or a hybrid between the nucleic acid probe and the target nucleic acid on the chip is in principle formed as a monolayer of molecules, such analysis basically requires a quite high sensitivity of surface analysis techniques.

While such highly sensitive surface analysis techniques known in the art include a method for labeling the nucleic acid probe and/or the target nucleic acid with an isotope, this method is not always commonly used since it is complex and dangerous while requiring special facilities and equipment.

Labeling the nucleic acid probe and/or the target nucleic acid with a fluorescent substance is considered to be an option. While a fluorescence hybridization method for labeling the target nucleic acid with a fluorescent substance is widely known in the art, this method involves many problems such as unstability of fluorescent pigments, quenching and nonspecific adsorption of the fluorescent pigment on the surface of the substrate. Therefore, these problems should be solved before quantitative determination of the nucleic acid probe and hybrid.

While the other high sensitivity surface analysis methods that are generally used include an ATR method using FT-IR (Fourier Transform IR spectroscopy) and XPS (X-ray Photoelectron Spectroscopy), these methods are not always sensitive enough for using for a quantitative analysis of the hybrid on the nucleic acid chip, and for imaging of the nucleic acid chip. In particular, when a commonly used glass plate is used as the substrate of the nucleic acid chip, there arise problems such as absorption of IR light by the glass plate in FT-IR (ATR), and charge up in XPS. Therefore, these methods cannot be considered to be effective methods.

U.S. Patent No. 5,821,060 discloses a DNA detection method by laser resonance ionization (RIS: Resonance Ionization Spectroscopy) as another high sensitivity surface analysis method. A target element is ionized and detected by irradiating a laser beam having a wavelength corresponding to the ionization energy of the target element emitted from the surface of the sample. While a method using a laser beam and a method using ions have been disclosed for permitting elements to be emitted from the surface of the sample, these methods involve a problem that only specified elements can be detected.

Dynamic Secondary Ion Mass Spectroscopy (Dynamic-SIMS) is another option of the high sensitivity surface analysis method. However, little information of the chemical structure is obtained from mass spectra since organic compounds are decomposed to small fragment ions or particles in the process for forming the secondary ions in this method, which is not suitable for the analysis of organic substances such as nucleic acid related substances.

On the contrary, the Time-of-Flight Secondary Ion Mass Spectrometry (TOF-SIMS) is used as an analytical method for investigating what kinds of atoms or molecules are existing on the uppermost surface of a solid sample, and has the following features: detection ability of minute components as small as 10⁹ atoms/cm² (corresponding to 1/10⁵ of the uppermost atomic monolayer); availability for both organic substances and inorganic substances; measurability of all atoms and compounds existing on the surface; and capability of secondary ion imaging from the substances existing on the surface of the sample.

The principle of the method will be briefly described hereinafter.

Constituting components on the surface are emitted in vacuum by a sputtering phenomenon by irradiating a high-speed ion (primary ion) beam onto the surface of the solid sample in high vacuum. Positively or negatively charged ions (secondary ions) emitted by irradiation are converged in one direction by an electric field, and are detected at a position a given distance apart. While the secondary ions having various masses are depending on the surface composition of the sample emitted by sputtering, the masses of the emitted secondary ions can be analyzed by measuring the time lapse (time-of-flight) from emission to detection of the secondary ions, since a lighter ion fly with larger velocities while a heavier ion fly with smaller velocities.

A little information on the chemical structure is obtained from mass spectra in usual dynamic-SIMS since organic compounds are decomposed to small fragment ions or particle by ionization as described above. However, the dose of the irradiated primary ions is so small in TOF-SIMS that the organic compounds are ionized while maintaining the chemical structures to enable the structure of the organic compound to be determined from the mass spectra. Since only the secondary ions generated at the uppermost surface of the solid sample are emitted in vacuum, information on the uppermost surface (with a depth of several Å) of the sample may be obtained.

The TOF-SIMS apparatus is roughly categorized into two types of sector type and reflectron type. One of the differences between these analysis methods is an electrical grounding method of a holder for fixing the analyzed sample. While the apparatus of the sector type is constructed so that the secondary ions are guided to the mass spectrometer by applying several kilovolts of positive or negative voltage on the holder in the apparatus of the reflection type, the holder is grounded, and the secondary ions are guided to the mass spectrometer by applying several to several tens kilovolts of positive or negative voltages on a secondary ion emitting electrode.

While positive primary ions are often used in the TOF-SIMS method, positive secondary ions and negative secondary ions are emitted irrespective of the polarity of the primary ions. The secondary electrons are generated by irradiation of the primary ions under general conditions of measurements irrespective of the polarity of the primary ions, and the amount of the generated secondary electrons are larger than the amount of the primary ions. Consequently, the surface of the analyzed sample tends to be positively charged to arise a defective measurement when electrification is in excess (so-called charge-up phenomenon). Positive electrification seems to be maximum when the negative secondary ions from an insulator are measured using an apparatus of the sector type considering the construction of the apparatus (because all of the secondary electrons generated are directed toward the secondary ion emitting electrode on which the positive voltage is applied).

Most of the apparatus of the sector type and reflectron type are equipped with a pulse electron gun for neutralizing positive electrification as described above. Specifically, electrification is neutralized by the electron gun by irradiating the analyzed sample with an electron beam from the pulse electron gun for a given time during a period from irradiation of the primary ions (sub- to several nanoseconds of pulses) and measurement of the time-of-flight of the positive or negative secondary ions to the succeeding irradiation of the secondary ions. Application of the voltage to the sample holder in the apparatus of the sector type, and application of the voltage to the secondary ion-emitting electrode in the apparatus of the reflectron type are suspended during the irradiation time of the electron beam to the analyzed sample, and the holder or electrode is grounded.

Although the positive electrification is relaxed (or quenched) by this method to enable the insulator to be analyzed, the margin for neutralizing electrification becomes narrowest because positive electrification tends to be the largest by the same reason as described above when the negative secondary ions are measured in the measurement of the insulator using the apparatus of the sector type. Anyhow, using the apparatus of the reflectron type comprising the electrically grounded sample holder is (usually) more advantageous than using the apparatus of the sector type for avoiding the charge-up phenomenon. When the conductivity of the analyzed sample, for example a glass, is low (in other words, resistivity or permittivity is high), the apparatus of the reflectron type may be suitable for the measurements.

Since the TOF-SIMA method is a highly sensitive measuring method, irrespective of the type of the apparatus used such as the reflectron type or the sector type, oligonucleotides formed as a molecular monolayer on, for example, a gold substrate on which the influence of charge-up is small may be analyzed. (Proceeding of the 12^{th} International Conference on Secondary Ion Mass Spectrometry 951, 1999). This literature describes the analytical results of DNA and PNA +immobilized on a substrate by the TOF-SIMS. According to this report, examples of the fragment ions detected by the TOF-SIMS method include PO₂⁻ and PO₃⁻ ions derived from phosphate backbones, and (thymine-H)⁻ ions derived from bases in the DNA probe, and (thymine-H)⁻ ions in the PNA probe.

However, there arise the following problems that formation/unformation of hybrids of the target DNA cannot be specifically detected by the following two reasons when obtaining desired gene information is attempted by detecting the target DNA by the TOF-SIMS method using generally used DNA chips:
(1) only a quite thin layer in the vicinity of the surface is detected by the TOF-SIMS method; and
(2) the fragment ion species generated from the probe DNA and target DNA are the same with each other.

For solving these problems, PNA is immobilized on a solid phase as a prove to form a hybrid between the PNA and the target nucleic acid (J. C. Feldner et al., SIMS XIII International Conference; 11 November, 2001, Nara). According to the method, the hybrid is confirmed to be formed between the PNA probe and the target nucleic acid by detecting the fragment ions from the phosphate backbone, since the peptide-nucleic acid has no phosphate backbone although the base of the peptide nucleic acid is the same as that of DNA.

However, acquiring gene information using the chip having the peptide-nucleic acid as a probe is not practical due to its high preparation cost, since the peptide-nucleic acid is expensive. Detection is relatively easy by using DNA as a probe since PO₂⁻ and PO₃⁻ ions have relatively high efficiency and parent structure of PO₂⁻ and PO₃⁻ ions are bonded to all the nucleotides. However, detection of the fragment ions of the base is often relatively difficult due to the disadvantageous number and relatively low ionization efficiency of the fragment ions, when four kinds of bases are randomly contained in the nucleic acid probe including the cases using PNA as the probe. Accordingly, a method for labeling the target nucleic acid capable of detection of the fragment ions with higher detection and quantitatively detecting efficiency over the foregoing art have been desired.

Japanese Patent Application Laid-Open No. 61-11665 discloses a nucleic acid base detector for detection of the fragment ions, wherein non-metallic elements such as S, Br and I, or metallic elements such as Ag, Au, Pt, Os and Hg are introduced into nucleic acid fragments separated by electrophoresis, liquid chromatography or high speed gel filtration depending on the molecular weight of the fragments, and these elements are identified by atomic absorption spectroscopy, plasma emission spectroscopy or mass spectroscopy. However, there are no detailed descriptions of the mass spectrometer as well as the method for introducing the halogen atoms in the nucleic acid.

On the other hand, the matrix itself on which nucleic acid probe regions are specifically formed should be analyzed from the point of view of quantitatively determining the nucleic acid hybrid on the chip. However, there are often no methods for locating the matrix (the spot on which discharged DNA is bonded) on the substrate when a solution of a previously synthesized DNA probe is discharged and immobilized on the surface of substrate by an ink-jet method, which is a method for producing the DNA chip described in Japanese Patent Application Laid-Open No. H11-187900. It is desirable in this case to analyze the fragment ions in an imaged spot after imaging the probe or hybrid in the spot by the TOF-SIMS method. However, no such methods are described in Japanese Patent Application Laid-Open No. H11-187900. Although Japanese Patent Application Laid-Open No. 61-11665 has mentioned the mass spectroscopic method as described above, there are no descriptions at all on the imaging method of the hybrid on the chip using the TOF-SIMS method and quantitative analysis of the target nucleic acid.

Accordingly, a novel method for detecting the probe and/or the target substance have been desired, whereby the detection efficiency is further improved over the foregoing art, and quantitative detection of the fragment ions are made possible.

### SUMMARY OF THE INVENTION

An object of the invention is to provide a method of analyzing a probe carrier that is capable of more precisely analyzing the state of probes immobilized on the probe carrier, for example, imaging of the locations of the probes and quantitative analysis thereof.

Another object of the invention is to provide a method of analyzing a measuring sample that is capable of accurate imaging of disposing locations or quantitative analysis of a complex between a probe formed on a measuring sample obtained by a reaction of a probe carrier with a sample and a target substance, more specifically a hybrid formed between a nucleic acid probe and a target nucleic acid.

The method of the invention is applicable to substances which recognize each other and form a complex, either one of which can be immobilized on a carrier, and into either one or both of which a marker having a high ionization efficiency, for example, halogen atoms can be introduced as a marker. Examples of such complex forming substances include proteins such as antigens, antibodies and enzymes, an enzyme and a substrate specifically binding to the enzyme, or mutually complementary nucleic acids.

The inventors have investigated the problems involved in imaging a hybrid between a nucleic acid probe and a target nucleic acid at a region having a relatively large area on a carrier having a relatively large resistivity, and in quantitatively determining the hybrid, using the TOF-SIMS.

In an aspect, the invention provides a method of detecting at least one of a probe and a target substance capable of specifically binding to the probe disposed on a substrate, the method comprising the steps of preparing a substrate having at least one of a probe and a target substance specifically bonded to the probe disposed on a surface thereof; and measuring the surface of the substrate by the Time-of-Flight Secondary Ion Mass Spectrometry (TOF-SIMS), wherein the probe and/or the target substance is labeled with a marker substance capable of forming a fragment ion that is not formed by fragmentation of the at least one of the probe and the target substance.

According to the invention, it is possible to effect imaging of disposing locations or quantitative analysis of probes immobilized on a carrier as a probe carrier and/or a complex formed between the probe and a target substance (a hybrid between a nucleic acid probe and a target substance when the target substance is a nucleic acid) with the probes or the hybrid being immobilized on the carrier.

In another aspect, the invention provides a method comprising reacting a sample with a probe carrier having a number of probe-immobilized regions disposed independently in a matrix pattern on a carrier and analyzing an analysis sample obtained by the reaction, wherein a target substance in the sample capable of specifically binding to the probe is labeled with a halogen atom and formation/unformation of a complex obtained by the reaction between the probe and the target substance is detected by measuring the halogen atom by the Time-of-Flight Secondary Ion Mass Spectrometry (TOF-SIMS).

According to the invention, it is possible to effect, for example, imaging of disposing locations or quantitative analysis with accuracy of formation/unformation of a complex between a probe formed in a measuring sample obtained by reacting a sample with a probe-immobilized probe carrier and a target substance (a hybrid between a nucleic acid probe and a target substance when the target substance is a nucleic acid) with the probes or the hybrid being immobilized on the carrier.

In a different aspect, the invention provides a method of analyzing a probe carrier having a number of probes disposed in a matrix pattern in a probe-immobilized region on the carrier by the Time-of-Flight Secondary Ion Mass Spectrometry, wherein the probes are labeled with halogen atoms and fragment ions of the halogen atoms are detected to analyze the state of probes.

According to the construction of the invention, the analysis of the state of probes immobilized on the probe carrier, for example imaging of disposing locations and quantitative analysis thereof can be more accurately performed.

Specifically, the construction above permits imaging and quantitative analysis of the nucleic acid probe at the same time by analyzing the halogen atoms of the halogen labeled nucleic acid on the probe carrier by the Time-of-Flight Secondary Ion Mass Spectrometry.

In a further different aspect, the invention provides a method of analyzing a nucleic acid chip comprising a plurality of nucleic acid probes disposed in a matrix pattern on a substrate, the method comprising the steps of hybridizing the nucleic acid probes with target nucleic acids in a sample to form hybrids; and simultaneously analyzing the nucleic acid probes and the target nucleic acids in the state of the hybrids, wherein the nucleic acid probes and the target nucleic acids are labeled with different marker substances of prescribed numbers and then analyzing the individual marker substances by the Time-of-Flight Secondary Ion Mass Spectrometry, thereby analyzing the labeled nucleic acid probes and the labeled target nucleic acids.

In the above method, it is preferable that the marker substance is neither a substance constituting the nucleic acid probe, nor a substance constituting the target nucleic acid. Specifically, it is preferable to select a substance capable of generating secondary ions that are distinctly distinguishable from secondary ions derived from the substance constituting the nucleic acid prove, and from secondary ions derived from the substance constituting the target nucleic acid.

According to the analysis method so constructed as described above, the probe nucleic acid and the target nucleic acid hybridized on the nucleic acid chip are previously labeled with different marker substances, for example halogen atoms. Consequently, the probe nucleic acid and the target nucleic acid can be independently imaged while independently quantifying them based on the measurement of the secondary ions derived from differently labeled marker substances using the Time-of-Flight Secondary Ion Mass Spectrometry.

### BRIEF DESCRIPTION OF THE DRAWINGS

Fig. 1A is a view showing the result of imaging of sequence No. 1 using ⁷⁹Br⁻ ions in Example 2;
Fig. 1B is a view showing the result of imaging of sequence No. 1 using ⁸¹Br⁻ ions in Example 2;
Fig. 2A is a view illustrating a method for continuous pattern irradiation with primary ions in a spot fashion for imaging;
Fig. 2B is a view illustrating a method for non-continuous pattern irradiation with primary ions in a spot fashion for imaging, the numbers given in Fig. 2B showing an irradiation order of the irradiation spot;
Fig. 3A is a view showing the result of imaging of sequence No. 1 using ⁷⁹Br⁻ ions in Example 2;
Fig. 3B is a view showing the result of imaging of sequence No. 1 using ⁸¹Br⁻ ions in Example 2;
Fig. 3C is a view showing the result of imaging of sequence No. 2 using ⁷⁹Br⁻ ions in Example 2;
Fig. 3D is a view showing the result of imaging of sequence No. 2 using ⁸¹Br⁻ ions in Example 2;
Fig. 3E is a view showing the result of imaging of sequence No. 3 using ⁷⁹Br⁻ ions in Example 2;
Fig. 3F is a view showing the result of imaging of sequence No. 3 using ⁸¹Br⁻ ions in Example 2;
Fig. 4A is a view showing the result of imaging using F⁻ ions in Example 2;
Fig. 4B is a view showing the result of imaging using ⁷⁹Br⁻ ions in Example 2;
Fig. 4C is a view showing the result of imaging using ⁸¹Br⁻ ions in Example 2;
Fig. 5 is a graphical representation showing plots of the measured values of marker F⁻ ions of the nucleic acid probe, and marker ⁷⁹Br⁻ ions and ⁸¹Br⁻ ions of the target DNA, respectively, after hybridization against the nucleic acid probe concentration in the nucleic acid probe solution to be spotted on the nucleic acid chip based on the results of quantitative analysis in Example 2; and
Fig. 6 is a graphical representation showing plots of the measured values of marker F⁻ ions of the nucleic acid probe, and marker ⁷⁹Br⁻ ions and ⁸¹Br⁻ ions of the target DNA, respectively, after hybridization against the target DNA concentration of the sample solution to be hybridized with the nucleic acid probe on the nucleic acid chip based on the results of quantitative analysis in Example 3.

### DESCRIPTION OF THE PREFERRED EMBODIMENTS

In the invention, the state of probes immobilized on a probe carrier or a target substance specifically bonded to the probes, for example, the location or quantity thereof is analyzed using probe and/or the target substance labeled with a marker substance capable of generating fragment ions, which are not generated by fragmentation of the probe or target substance.

Specifically, a substance having a high ionization efficiency, favorably ion fragments derived from halogen atoms, may be detected by the Time-of-Flight Secondary Ion Mass Spectrometry (TOF-SIMS).

In other words, probes on the probe carrier and/or a target substance is preferably labeled with a prescribed number of halogen atoms for imaging and analysis of the probe carrier using TOF-SIMS, and fragment ions of the halogen atom are detected and analyzed by TOF-SIMS.

The probes and/or the target substance is labeled with a marker substance capable of generating fragment ions that are not generated by fragmentation of the probes and/or the target substance.

The methods available are as follows:
(1) The target substance is labeled preferably with a prescribed number of the halogen atoms for imaging and analysis of a hybrid using TOF-SIMS, and the fragment ions of the halogen atoms are detected by TOF-SIMS.
(2) The state of probes immobilized on the carrier, for example the location and quantity thereof, is analyzed by detecting the fragment ions derived from the halogen atoms labeled on the probe by the Time-of-Flight Secondary Ion Mass Spectrometry (TOF-SIMS). In other words, the probe on the probe carrier is labeled preferably with a prescribed number of the halogen atoms for imaging and analysis of the probe carrier using TOF-SIMS, and the fragment ions of the halogen atoms are detected and analyzed by TOF-SIMS.
(3) A probe nucleic acid and the target nucleic acid, respectively, are labeled with different marker substances of prescrived number for imaging and quantitative analysis of the hybrid formed using TOF-SIMS, and fragment ions of mutually different substances are detected and analyzed by TOF-SIMS. The marker substance available is preferably different from the constituting elements such as the probe nucleic acid, the substances constituting the target nucleic acid and the nucleic acid, since the secondary ions derived from the marker substance can be distinctly extinguished from the fragment ions derived from the nucleic acid. In addition, the probe nucleic acid and the target nucleic acid, respectively, are preferably labeled with prescribed numbers of the marker substances in order to quantitatively determine respective nucleic acids.

Examples of the marker substances are, although not restrictive, halogen atoms such as fluorine, chlorine, bromine and iodine.

Improvements of detection sensitivity can be expected by using fragment ions of the halogen atoms having relatively high ionization efficiency, and the effects of charge-up can be excluded by reducing the intensity of the primary ion in proportion to the degree of reduction of the intensity. Accordingly, large area imaging on a high resistivity substrate is possible by combining with a method to be described hereinafter.

The marker substance used in the invention preferably has a high ionization efficiency than the fragments contained in the probe and the target substance in TOF-SIMS analysis. When both the probe and the target substance are labeled, they are preferably labeled with different marker substances from each other.

The problems arising from detecting only the nucleic acid's own fragment ions as described above can be solved by labeling the nucleic acid with a prescribed number of halogen atoms particularly when the method of the invention is applied to the nucleic acid, and the nucleic acid can be analyzed with improved quantitative accuracy.

Further, the problems arising from detecting only the nucleic acid's own fragment ions as described above can be also solved by labeling the nucleic acid with a prescribed number of the halogen atoms, and the nucleic acid can be analyzed with improved quantitative accuracy. The number of labeling with the halogen atoms is not particularly restricted. Any positions and methods for labeling are available, so long as they are applicable and do not inhibit complexes from being formed (hybrid complex (hybridization) when the target substance is a nucleic acid) between the probe and the target substance from being formed thereafter.

Practically, one position is labeled in one nucleotide molecule. Accordingly, the prescribed number of the halogen atoms used as a marker is desirably an arbitrary number from 1 to the number of the nucleotides in the probe nucleic acid and the target nucleic acid. For example, the more desirable number is 1 to 5 when the nucleic acid is a synthetic oligonucleotide considering the labor and cost of labeling, and the degree of ionization efficiency of the halogen atoms.

When introduction of the marker is attempted by taking advantage of an enzymatic elongation reaction such as a PCR method, the number of the introduced marker is restricted due to steric hindrance when the marker is a relatively large molecule such as a fluorescent pigment. On the contrary, the halogen atom induces substantially no steric hindrance. For example, all the same kind of bases (for example adenine) can be labeled in an elongation product by using a nucleic acid base unit substituted with halogen atoms for the elongation reaction. Accordingly, selecting the halogen atom as the marker is desirable for enabling the number of the markers to be quantitatively determined in addition to the sensitivity and method for introducing the marker.

The secondary ions of the fluorine, chlorine, bromine and iodine atoms can be detected with high sensitivity in the analysis by TOF-SIMS. Since the four kinds of the halogen atoms can be introduced in the target nucleic acid according to the method to be described hereinafter, these halogen atoms may be favorably utilized in the invention.

The probe immobilized on the carrier is able to recognize a specific target substance and to form a complex with the target substance. When the target substance is a nucleic acid, the probe can be specifically bonded to the target nucleic acid by a complementary sequence of the nucleic acid probe with the target nucleic acid. The probe immobilized on the carrier should be able to be specifically bonded to a specified target substance, and the method of the invention is principally applicable to not only the nucleic acid, but also to substances capable of labeling with halogen atoms, for example proteins such as antigens, antibodies and enzymes substrates, and substrates specifically bonded to the enzymes.

Any methods known in the art may be used for immobilizing the nucleic acid probe on the carrier in the invention. In an example of the probe immobilized on the carrier, a binding site with the carrier is formed with interposition of a linker, if necessary, at a part of the nucleic acid probe comprising oligonucleotides having base sequences capable of hybridizing with the target nucleic acid, and the probe is linked to the surface of the carrier at binding sites with the carrier. The position of the binding site with the carrier so constructed as described above in the nucleic acid probe molecules is not particularly restricted, so long as a desired hybridization reaction is not impaired.

Independent regions immobilizing the probe, for example many dots, are arranged in a matrix pattern with a given space in the probe carrier of the invention. Such probe carrier includes a probe array, microchip nucleic acid chip and the like.

On the other hand, the probe has a structure capable of being bonded to the surface of the carrier, and the probe is desirably immobilized through this structure. Preferably, the structure of the probe capable of bonded to the surface of the carrier is formed by introducing at least one of organic functional groups such as an amino group, a thiol group, a carboxylic group, a hydroxyl group, an acid halide (haloformyl group; -COX), a halide group (-X), an aziridine group, a maleimide group, a succimide group, an isothiocyanate group, a sulfonyl chloride group (-SO₂Cl), an aldehyde group (formyl group; -CHO), a hydrazine group and an acetamide iodide group.

Immobilization of the probe by covalent bonds are possible by a treatment required for the surface of the carrier, or by a treatment for forming a maleimide group for the thiol group, an epoxy group, aldehyde group or N-hydroxysuccimide for the amino group, depending on the structure required for binding the probe on the carrier.

The probe is desirably bonded to the surface of the substrate by the covalent bond considering the stability of the probe.

Examples of the combination of the probe with the target substance include a combination between the nucleic acid probe and the target nucleic acid, and a combination capable of forming a complex selected from proteins such as antigens, antibodies and enzymes, and substrates capable of specifically binding to the enzyme.

The nucleic acid probes used in the invention are not particularly restricted, and any nucleic acid probes are available so long as they are able to recognize the target nucleic acid. However, the nucleic acid probe is desirably selected from DNA, RNA, PNA (peptide-nucleic acid), cDNA (complementary DNA), cRNA (complementary RNA) and PCR amplification products (from cDNA). Preferably, a nucleic acid probe comprising at least one of them may be immobilized on the carrier.

The target nucleic acid used in the invention is desirably DNA, RNA, PNA (peptide nucleic acid), cDNA (complementary DNA), cRNA (complementary RNA) and PCR amplification products (from cDNA) considering the method for labeling with the halogen atoms to be described hereinafter. A sample containing the target nucleic acid comprising at least one of them may be used for analysis. The target nucleic acid may be a synthetic nucleic acid, or a natural nucleic acid derived from animals, human, plants, microorganisms and the like.

The imaging method of the measuring sample (a probe carrier prepared by required treatments after allowing to react with a sample: the "nucleic acid chip" will be described hereinafter as a representative) of the invention comprises: sequentially irradiating the primary ions onto a portion on the surface of the nucleic acid surface having a given area as a pulse spot having a relatively smaller area than the area above; and analyzing the secondary ions emitted by the pulse irradiation by the Time-of-Flight Secondary Ion Mass Spectrometry for every pulse irradiation. For excluding the effect of charge-up, it is quite effective and desirable that the pulses of the primary ions are irradiated as a non-continuous pattern, and the results of the mass spectroscopic analysis obtained are reconstructed for imaging based on the pattern of non-continuous irradiation of the primary pulse.

It has been considered to be desirable that the area of the imaging region is, for example, larger than 300 µm × 300 µm or more considering the size of the spot, or the detection efficiency, when the nucleic acid chip is imaged and the nucleic acid on the nucleic acid chip is quantitatively analyzed by TOF-SIMS. However, the effect of charge-up becomes large for obtaining an image by sequential scanning (raster scanning) of the beam in a given direction as is used in television picture tubes, when the diameter of the primary beam is adjusted to 5 µm for obtaining a required resolution and the area of 300 µm × 300 µm is scanned with the beam on the substrate having a relatively high resistivity such as a glass that is frequently used as a substrate for preparing the DNA chip. Consequently, good images could not be obtained.

Accordingly, the primary ions are sequentially irradiated as a pulse spot to the portion having a given area on the surface of the nucleic acid chip so that the spot has a relatively smaller area than the area above, and the secondary ions emitted by pulse irradiation are analyzed by time-of-flight mass spectroscopy for every pulse irradiation in the invention. For excluding the effect of charge up, it is quite effective and desirable that the primary pulse is irradiated based on a non-continuous pattern, and the results of the mass spectroscopic analysis obtained are reconstructed based on the pattern of primary pulse irradiation.

Examples of the non-continuous pattern include a random pattern and a programmed non-continuous pattern.

Simple examples of the continuous pattern and non-continuous pattern are as follows.

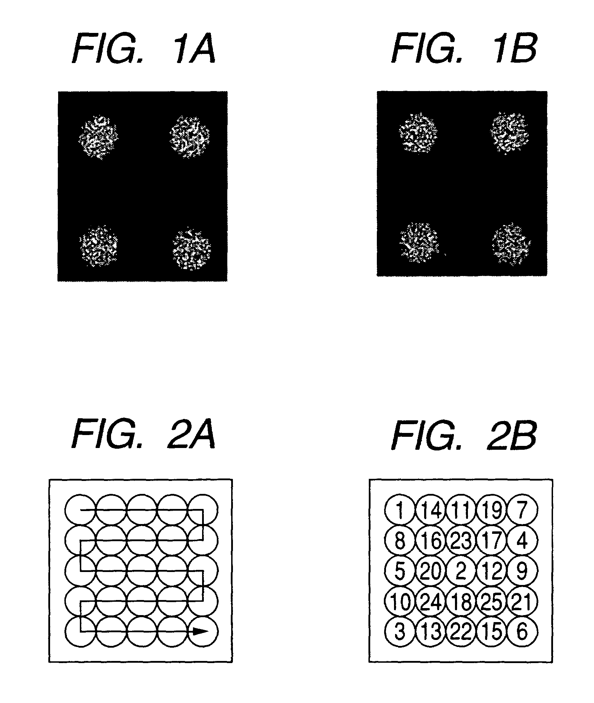

Fig. 2A shows an example of the continuous irradiation pattern, while Fig. 2B shows an example of the non-continuous irradiation pattern. Suppose that the primary ion pulse is irradiated on 5 × 5 spots each having a rectangular area, then a pattern obtained by sequentially irradiating all the spots from one spot to a neighboring spot is a continuous pattern as shown in Fig. 2A. On the other hand, a pattern obtained by sequentially irradiating all the spots from one spot to at least a non-neighboring spot is a non-continuous pattern.

It is possible to form the non-continuous pattern in a random order. However, since neighboring spots may be continuously irradiated by this method, a non-continuous pattern according to a special program can be formed. An algorithm may be appropriately employed for the desirable pattern so that the neighboring spots are not continuously irradiated, or the spots on neighboring columns and rows are not continuously irradiated.

Examples of the halogen atoms include fluorine, chlorine, bromine and iodine atoms. Fragment ions of the fluorine, chlorine, bromine and iodine atoms can be detected by TOF-SIMS. These four kinds of the halogen atoms can be introduced into the nucleic acid probe by the methods to be described hereinafter.

The nucleic acid probes in each matrix of the probe carrier may be labeled with different halogen atoms with each other.

When the sample is unpredictable whether it contains a target substance or not, the sample is labeled with the halogen atom at first, and a probe is allowed to react with the sample. Then, a complex is formed when the sample contains a target substance capable of being recognized by the probe, and the complex can be detected using the marker halogen atom.

The fragment ions of the fluorine, chlorine, bromine and iodine can be detected by TOF-SIMS. These halogen atoms can be efficiently utilized in the invention since the four kinds of the halogen atoms can be also introduced into the target nucleic acid by the methods to be described hereinafter.

While the method for introducing the halogen atom in the target nucleic acid is not particularly restricted, and an example of the method known in the art is to permit the halogen atom to be bonded to the nucleic acid base of the target nucleic acid, which can be favorably used in the invention. The halogen atom is desirably bonded at a position that does not inhibit a hybrid of the target nucleic acid from being formed when the target nucleic acid forms the hybrid with the nucleic acid probe. Such binding sites are the 5-position of the pyrimidine base and the 8-position of the purine base. However, it is not always required that the halogen atoms in all the nucleotide bases of the target nucleic acid are bonded to these positions.

While the method for introducing the halogen atom into the nucleic acid prove is not restricted, and the method well known in the art is to allow the halogen atom to be bonded to the nucleotide base of the nucleic acid probe. This method can be favorably used in the invention. It is desirable that the halogen atom is bonded to the position that does not inhibit a hybrid of the nucleic acid probe from being formed when the nucleic acid probe forms the hybrid with the target nucleic acid. Such binding sites are the 5-position of the pyrimidine base and the 8-position of the purine base. However, it is not always required that the halogen atoms in all the nucleotide bases of the nucleic acid probe are bonded to these positions.

In a practical method for introducing the halogen atom in the nucleic acid probe or target nucleic acid when the nucleic acid is a synthetic DNA, a synthetic unit binding the halogen unit, or 2'-deoxyribonucleoside-3'-phosphoroamidite represented by the following structural formula may be used for synthesizing the DNA using an automatic DNA synthesizer: 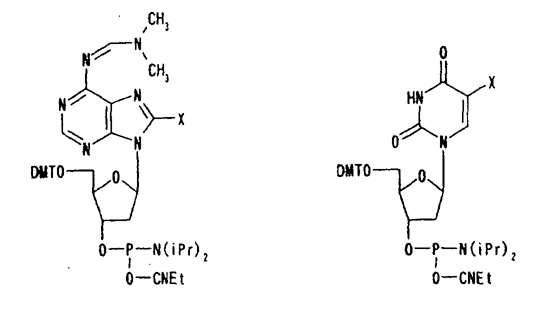 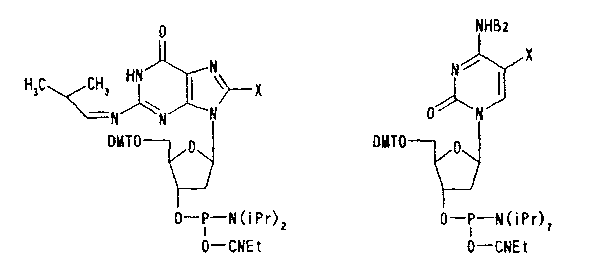 wherein X represents a halogen atom, DMTO represents a dimethoxytrityl group, iPr represents an isopropyl group, and CNEt represents a 2-cyanoethyl group.

When the nucleic acid is a synthetic RNA, on the other hand, a synthetic unit binding the halogen atom, or ribonucleoside-3'-phosphoroamidite, may be used for synthesizing the RNA using an RNA automatic synthesizer. Examples of such synthetic unit include the following compound: 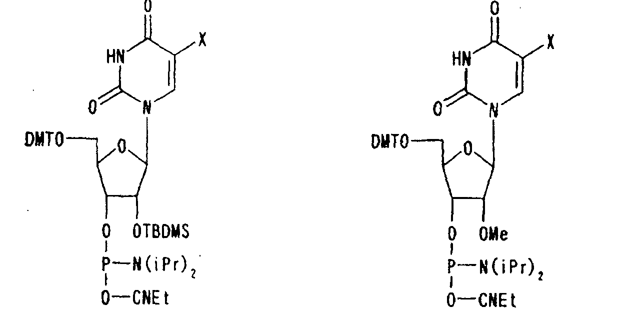 wherein X represents a halogen atom (F, Cl, Br or I), DMTO represents a dimethoxytrityl group, iPr represents an isopropyl group, CNEt represents a 2-cyanoethyl group, ME represents a methyl group, and TBDMS represents a t-butyldimethoxyxylyl group).

When the nucleic acid is a synthetic PNA, A synthetic unit binding the halogen atom, or a peptide analogue binding a nucleic acid base may be favorably used for synthesizing PNA using a PNA automatic synthesizer.

In an example for introducing the halogen atom when the nucleic acid is cDNA, 2'-deoxyribonucleoside-5'-triphosphate binding the halogen atom may be used for elongating a cDNA with a reverse transcriptase.

In an example for introducing the halogen atom when the nucleic acid is a DNA derived from a genome DNA, 2'-deoxyribonucleoside-5'- phosphate binding the halogen atom may be used for elongating the DNA with DNA polymerase. For introducing the halogen atom into the cRNA when the nucleic acid is cRNA, on the other hand, ribonucleoside-5'-triphosphate binding the halogen atom may be used for elongating the cRNA with RNA polymerase.

A PCR reaction, or a RT-PCR (reverse transcription PCR) reaction can be used for the elongation reaction. A marker may be introduced together with the nucleotide monomer used for the elongation reaction, or the marker may be introduced in the synthesis of a primer used for the reaction.

The nucleic acid probe or target nucleic acid species of the nucleic acid chip used in the invention are not particularly restricted, and DNA, RNA, PNA (peptide-nucleic acid), cDNA (complementary DNA), cRNA (complementary RNA), oligodeoxynucleoside, oligoribonucleotide and the like may be used.

Other examples of the target substance available in the analysis method of the invention include metals such as Au, Ag, Cu, Ni, Co, Cr, Al, Ta, Pt, Pd, Zn, Sn, Ru and Rh, and metal complexes thereof including organic (metal) complexes. The method described in Science, Vol. 262, 1025, 1993 may be used, for example, for introducing the organic metal complex.

### [Examples]

The invention will be described in detail with reference to examples. Although these examples constitute a part of the best mode for carrying out the invention, the invention is not restricted to these examples.

### (Example 1) Preparation of Nucleic Acid Probe Chip

The nucleic acid probe chip was prepared according to Japanese Patent Application Laid-Open No. H11-187900.

### (1) Cleaning of substrate

Synthetic quartz substrates (25.4 mm × 25.4 mm × 1 mm) were placed on a rack and soaked in a ultrasonic wave detergent (GPII produced by Blanson) diluted to 10% with water overnight. The substrate was washed with the detergent for 20 minutes using a ultrasonic wave followed by washing with water to remove the detergent. After rinsing with pure water, the substrate was further treated with the ultrasonic wave for 20 minutes in a vessel filled with pure water. Then, the substrate was soaked in a 1N aqueous sodium hydroxide solution previously heated at 80°C for 10 minutes, followed by washing with water and pure water to subject the substrate to the next step.

### (2) Surface treatment

A 1% by weight aqueous solution of a silane coupling reagent binding amino groups (N-β-(aminoethyl)-γ-aminopropyltrimethoxy silane: KBM603 produced by Shin-Etsu Chemical Co.) was stirred for 2 hours at room temperature to hydrolyze intermolecular methoxy groups in the silane compound. After soaking the substrate obtained in (1) for 1 hour at room temperature, the substrate was washed with pure water and dried by blowing nitrogen gas onto both surfaces of the substrate. Then, the substrate was baked for 1 hour in an oven heated at 120°C to finally introduce the amino group on the surface of the substrate.

Subsequently, 2.7 mg of N-maleimidecaproyloxysuccimide (EMCS produced by DOJINDO LABORATORIES.) was dissolved in a 1:1 solution of dimethylsulfoxide (DMSO) and ethanol in a concentration of 0.3 mg/ml. The quartz substrate after subjecting to the silane coupling treatment was soaked in this EMCS solution for 2 hours at room temperature, and the amino group bonded on the surface of the substrate was allowed to react with the succimide group in the EMCS solution by the silane coupling treatment. The maleimide group derived from EMCS is bonded on the surface of the substrate by this treatment. The substrate after pulling up from the EMCS solution was sequentially washed with the mixed solution of DMSO and ethanol, and ethanol, followed by drying by blowing nitrogen gas.

### (3) Synthesis of probe DNA

A single strand nucleic acid (40-mer of dT) of sequence No: 1 was synthesized by requesting to a DNA synthesis company (BEX). A thiol group (SH) was introduced in the 5'-terminal of the single strand DNA of sequence No. 1 by using a thiol modifier (Glen Research) in the synthesis step. Deprotection and recovering of DNA were performed by a usual method, and the product was purified by HPLC (High Performance Liquid Chromatography). A series of steps from synthesis to purification were requested to the synthesis company.

[Sequence No: 1]
5′ HS-(CH2)6-O-PO2-O-TTTTTTTTTT TTTTTTTTTT
TTTTTTTTTT TTTTTTTTTT 3′

### (4) Discharge of DNA by thermal jet printer and binding to substrate

The single strand DNA of sequence No: 1 was dissolved in a solution containing 7.5% by weight of glycerin, 7.5% by weight of urea, 7.5% by weight of thiodiglycol and 1% by weight of acetylene alcohol (trade name: Acetylenol EH produced by Kawaken Fine Chemical Co.) in a concentration of 8 µm. A printer head BC-50 (manufactured by Canon Inc.) for a bubble jet printer BJF-850 (manufactured by Canon Inc.) using a bubble jet method as a kind of thermal jet methods was reassembled so that several hundred microliters of the solution is discharged. This head was mounted on a discharge drawing machine reassembled so as to be able to discharge on the quartz substrate. Injected in a reassembled tank of the head was several hundred microliters of the DNA solution, and the solution was spotted on a substrate treated with EMCS using the discharge drawing machine. The discharge volume during spotting was 4 picoliter/drop, and the solution was discharged at 200 dpi, or 127 µm pitch, in a 10 mm × 10 mm range of spotting at the center of the substrate. The diameter of the dot spotted under the condition above was about 50 µm.

After completing to spot, the substrate was allowed to stand still in a moisturizing chamber for 30 minutes to allow the maleimide group on the surface of the glass plate to react with the thiol group at the terminal of the nucleic acid probe. After washing the substrate with pure water, it was stored in a 50 mM phosphate buffer solution (pH = 7.0, named as solution A hereinafter) containing 1M of NaCl.

### (Example 2) Imaging and Analysis by Hybridization and TOF-SIMS

### (1) Synthesis of model target nucleic acid

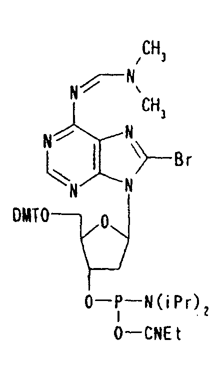

[Sequence No: 2]

5′ A(Br) A(Br) A(Br) A(Br) A(Br) AAAAA AAAAAAAAAA

AAAAAAAAAA AAAAAAAAAA 3′

A model terget nucleic acid (40-mer of dT; sequence No: 2) labeled with five bromine atoms was synthesized (BEX). Five bromine labeled nucleotides at the 5'-end were introduced during the synthesis with an automatic synthesizer using 8-bromo-3'-deoxyadenosine phosphoroamidite represented by the structure above. Deprotection and recovering of the DNA was performed by a usual method, and the product was purified by HPLC. A series of steps from the synthesis to purification was requested to a synthesis company. A(Br) in the sequence denotes bromine labeled deoxyadenosine. The 8-position of adenine as a marker position is known not to inhibit hybridization.

### (2) Blocking and hybridization

The chip prepared in Example 1 was soaked in solution A containing 2% bovine thymus albumin (BSA). After blocking the surface of the chip (for non-specific adsorption of nucleic acids and the like), the chip was soaked in solution A in which the target nucleic acid of sequence No: 2 is dissolved in a concentration of 50 nM for hybridization at 45°C for 15 hours. Then, after rinsing the chip with pure water (at room temperature), it was dried by blowing nitrogen gas, followed by storage in a vacuum desiccator before used for TOF-SIMS.

### (3) Analysis by TOF-SIMS

The DNA chip after hybridization was imaged and analyzed using the TOF-SIMS IV apparatus manufactured by ION TOF Co.

The conditions of the apparatus were as follows:
(Primary ion)
   primary ion: 25kV, Ga⁺ random scanning mode
   primary ion pulse frequency: 2.5 kHz (400 µsec/shot)
   primary ion pulse width: 1 ns
   primary ion beam diameter: 5 µm
(Secondary ion) imaging by reconstruction of the primary ion irradiation pattern
   secondary ion detection mode: negative
   measuring region: 300 µm × 300 µm
   pixel number of secondary ion: 128 × 128 integration times: 256

### (4) Results

Figs. 1A and 1B show the result of imaging on bromine ions from the data obtained after an analysis of the hybridized DNA chip in (2) by TOF-SIMS under the conditions above. Figs. 1A and 1B were obtained using ⁷⁹Br⁻ ion and ⁸¹Br⁻ ion.

**Table 1**

| Ionic species | Counts |
|---|---|
| ⁷⁹Br⁻ ion | 1250 |
| ⁸¹Br⁻ ion | 1285 |
| Total | 2663 |

Table 1 shows counts of each one spot obtained from Figs. 1A and 1B. Figs. 1A and 1B, and Table 1 show that the spot of the bromine labeled target DNA forming a hybrid with the nucleic acid probe on the DNA chip may be quantitatively analyzed by Br imaging. (Example 3) Imaging and Analysis of Bromine Labeled Target DNA Derived from Genome

### (1) Preparation of nucleic acid chip for detecting target DNA derived from genome

Nature Biotechnology Vol. 18, 483, 2000 describes preparation of an oligonucleotide chip for detecting exon 7 of the genome DNA of two cell lines HSC4 and HSC5 of oral cavity epidermoid carcinoma, and detection of fluorescence labeled DNA derived from the exon.

The oligonucleotide chip was prepared in this example according to the method above to synthesize a DNA using bromine in place of a fluorescent marker, followed by hybridization using the DNA.

The actual procedure thereof will be described below.

### (Synthesis of DNA probe and preparation of chip)

[Sequence No: 3]
5′ HS-(CH2)6-O-PO2-O-GATGGGCCTCCGGTTCAT 3′

The DNA of sequence No: 3, having a base sequence complementary to a part of the base sequence of exon 7 of HSC4 (the part containing codon No. 248) above and carrying a thiol group at the 5' terminal for binding to the substrate, was synthesized as in Example 1, and a DNA chip was prepared by the same method as in Example 1 using the DNA.

### (2) Synthesis of bromine labeled DNA derived from genome

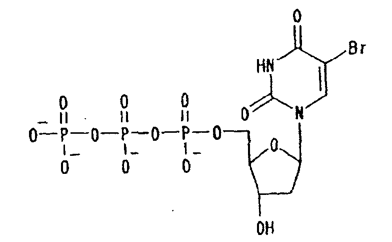

[Sequence No: 4]
E7S: 5'-ACTGGCCTCATCTTGGGCCT-3' (exon 7, sense)

[Sequence No: 5]
E7A: 5′-TGTGCAGGGTGGCAAGTGGC-3′ (exon 7, anti-
sense)

5-bromo-2'-deoxyuridine triphosphate (Br-dUTP)

An exon 7 portion was synthesized from the genome of HSC4 by a PCR reaction using PCR primers of sequence Nos: 4 and 5. Subjected to PCR amplification by repeating 40 cycles of 94°C (30 seconds) and 60°C (45 seconds) were 50 µL of PCR mixtures containing 20 ng of genome DNA and 0.4 µM each of sense and ant-sense primers. The nucleotides obtained were designed to have a chain length of 171 nucleotides.

Subsequently, 0.2 µm of anti-sense primer (sequence No: 4) and 10 µm of 5-bromo-2'-deoxyuridine triphosphate (Sigma-Aldrich Japan Co.) as a kind of the bromine labeled nucleotide having the structure shown above were subjected to ssPCR (single strand PCR) using a part of the amplification products as primers. The PCR cycles were 25 cycles of 96°C (30 seconds), 50°C (30 seconds) and 60°C (4 minutes). The bromine labeled single strand DNA obtained was purified by gel filtration.

### (3) Blocking and hybridization

After blocking the chip prepared in (1) above by the same method as in Example 1, the chip was rinsed with pure water and used for hybridization below. The chip after blocking was soaked in a SSPE solution (0.9M NaCl, 60 mM NaH₂PO₄, 6 mM EDTA) containing 20% of formamide six times followed by heating at 80°C for 10 minutes. The solution contained the DNA derived from genome synthesized as (2) above. Then, the chip was subjected to hybridization at 45°C for 15 hours, followed by washing with the SSPE solution twice at 55°C. Then, the chip was gently rinsed with pure water (at room temperature) followed by drying by blowing nitrogen gas to store in a desiccator before using in TOF-SIMS.

### (4) Imaging and analysis by TOF-SIMS

The DNA chip after hybridization under the same condition as in Example 2 was imaged and analyzed by TOF-SIMS.

The numerical data obtained are shown in Table 2.

**Table 2**

| Ionic species | Counts |
|---|---|
| ⁷⁹Br⁻ ion | 542 |
| ⁸¹Br⁻ ion | 620 |
| Total | 1162 |

Table 2 shows that hybridization of the target DNA derived from the genome and labeled with bromine on the DNA chip can be quantitatively determined by TOF-SIMS.

Labeling of the cDNA derived from mRNA with the halogen atom, and imaging and analysis thereof by TOF-SIMS are also possible by approximately the same method as in this example.

### (Example 4) Analysis of Binding of Probe

The surface of the substrate was (1) washed and (2) treated by the same procedure in Example 1.

### (3) synthesis of nucleic acid probe DNA

Single strand nucleic acids with sequence Nos: 6 to 8 were synthesized by requesting to the DNA synthesis company (BEX). In the sequence, base T represents usual 2'-deoxythymidine, and U(Br) represents 5-bromo-2'-deoxyuridine, which were introduced in the synthesis step using the phosphoroamidite (Glen Research) shown below.

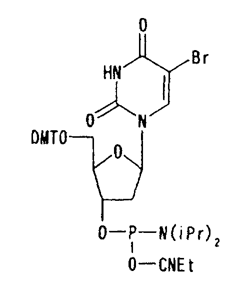

The terminal U(Br) was introduced using a (CPG) column (Glen Research) to which U(Br) shown below is immobilized. Bromine introduced the 5-position is known not to inhibit hybridization.

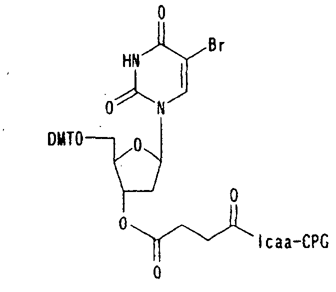

The thiol group was introduced to the 5'-terminal of DNA by using a thiol modifier (Glen Research) in the synthesis step. The DNA was deprotected and recovered by a usual method, HPLC was used for purification. A series of steps from the synthesis to purification were requested to the synthesis company.

[Sequence No: 6]

5′ HS-(CH2)6-O-PO2-O-TTTTTTTTTT-TTTTTTTTTT-

TTTTTTTTTT-TTTTTTTTTU(Br) 3′

[Sequence No: 7]

5′ HS-(CH2)6-O-PO2-O-TTTTTTTTTT-TTTTTTTTTT-

TTTTTTTTTT-TTTTTTTTU(Br)U(Br)U(Br) 3′

[Sequence No: 8]
5′ HS-(CH2)6-O-PO2-O-TTTTTTTTTT-TTTTTTTTTT-
TTTTTTTTTT-TTTTTU(Br)U(Br)U(Br)U(Br)U(Br) 3′

### (4) Discharge of DNA by thermal jet printer and binding to substrate

The single strand DNAs of sequence Nos: 6 to 8 were each dissolved at a concentration of 8 µM in a solution containing 7.5% by weight of glycerin, 7.5% by weight of urea, 7.5% by weight of thiodiglycol and 1% by weight of acetylene alcohol (trade name: Acetylenol EH produced by Kawaken Fine Chemical Co.).

Using the discharge drawing machine used in Example 1, 100 µL each of the DNA solutions was filled in the reconstructed tank, and the three sheets of the substrate treated with EMCS were mounted on the discharge drawing machine, and the three kinds of the DNA solutions were spotted on one sheet each of the three substrates. The discharge volume during spotting was 4 picoliter/drop, and the solution was discharged at 200 dpi, or 127 µm pitch, in a 10 mm × 10 mm range of spotting at the center of the substrate. The diameter of the dot spotted under the condition above was about 50 µm.

After completing to spot, the substrate was allowed to stand still in a moisturizing chamber for 30 minutes to allow the maleimide group on the surface of the glass plate to react with the thiol group at the terminal of the nucleic acid probe. Each substrate was washed with pure water, and was stored in pure water. Immediately before analysis by TOF-SIMS, the DNA bonded substrate (DNA chip) was dried by blowing nitrogen gas, and was further dried in a vacuum desiccator.

### (Example 5) Imaging and Analysis by TOF-SIMS

### (1) The DNA chips prepared in Example 4 were imaged and analyzed using TOF-SIMS IV apparatus manufactured by ION TOF Co. The conditions of the apparatus are summarized below:

(Primary ion)
   Primary ion: 25 kV Ga⁺, random scan mode
   Primary ion pulse frequency: 2.5 kHz (400 µsec/shot)
   Primary ion pulse width: 1 ns
   Primary ion beam diameter: 5 µm
(Secondary ion) imaging by reconstruction on the irradiation pattern of the primary ion
   Secondary ion detection mode: negative
   Measuring region: 300 µm × 300 µm
   Pixel number of secondary ion images: 128 × 128
   Integration time:

### (2) Results

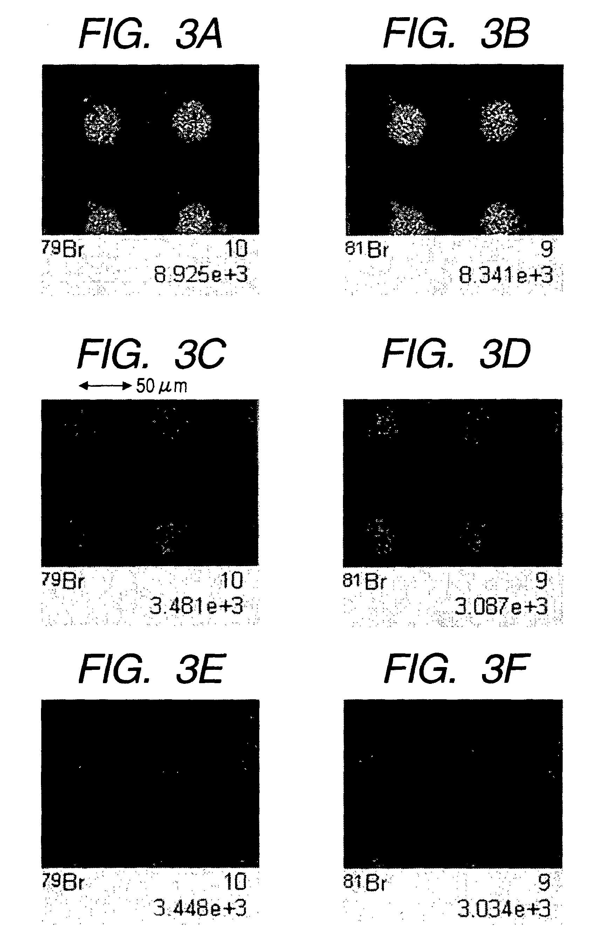

The DNA chips prepared in Example 4 were analyzed by the TOF-SIMS IV apparatus under the conditions above, and the bromide ion was imaged from the data obtained. The results obtained are shown in Figs. 3A to 3F. Figs. 3A and 3B are images of sequence No: 6, Figs. 3C and 3D are images of sequence No: 7, and Figs. 3E and 3F are images of sequence No: 8. Figs 3A, 3C and 3E are derived from ⁷⁹Br⁻ ion, while Figs 3B, 3D and 3F are derived from ⁸¹Br⁻ ion.

**Table 3**

| Ionic species | Counts | | |
|---|---|---|---|
| | Sequence No: 6 | Sequence No: 7 | Sequence No: 8 |
| ⁷⁹Br⁻ ion | 1342 | 805 | 273 |
| ⁸¹Br⁻ ion | 1321 | 800 | 224 |
| Total | 2663 | 1605 | 497 |

Table 3 shows the counts of one spot from each of Figs. 3A to 3F. Figs. 3A to 3F, and Table 3 show that imaging by bromine as a target substance of the spot of the bromine labeled DNA on the DNA chip as well as quantitative determination of bromine are possible, although is a relative value.

### (Example 6) Imaging and Analysis of Bromine Labeled DNA Chip Derived from Genome

### (1) Preparation of bromine labeled DNA chip derived from genome

DNA was synthesized according to the detection method of the fluorescence labeled DNA described in Nature Biotechnology Vol. 18, 438, 2000, cited in Example 3, wherein the marker was replaced from a fluorescence substance to bromine. Then, a DNA chip was prepared using the DNA according to the method described in Science Vol. 270, 467, 1995 (this reference relates to a method for preparing a cDNA chip).

An actual procedure of the method will be described below.

### (1) Synthesis of Bromine labeled DNA derived from genome

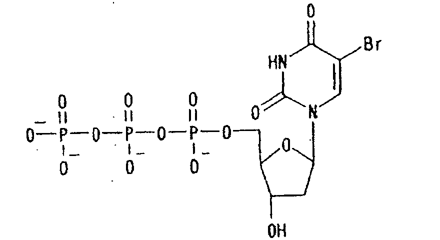

[Sequence No: 4]
E7S: 5'-ACTGGCCTCATCTTGGGCCT-3' (exon 7, sense)

[Sequence No: 5]
E7A: 5′-TGTGCAGGGTGGCAAGTGGC-3′ (exon 7, anti-
sense)

5-bromo-2'-deoxyuridine triphosphate (Br-dUTP)

The exon 7 part was synthesized from the genome of HSC4 by a PCR reaction using the PCR primers of sequence Nos: 4 and 5 used in Example 3 (common to HSC4 and HSC5: requested to BEX Research Co.).

A PCR mixture (50 µl) containing 20 ng of a genome DNA and 0.4 µM each of sense or anti-sense primers were amplified by PCR by repeating 40 cycles of 94°C (30 seconds) and 60°C (45 seconds). The DNA obtained was designed to have a chain length of 171 nucleotides.

Then, 0.2 µM of a sense primer (sequence No: 4) and 10 µM of 5-bromo-2'-deoxyuridine triphosphate (Sigma Aldrich Japan Co.) as a kind of bromine labeled nucleotide having the structure shown above was subjected to ssPCR (single strand PCR) using a part of the amplification product as a template. The PCR was performed by 25 cycles of 96°C (30 seconds), 50°C (30 seconds) and 60°C (4 minutes). The bromine labeled single strand DNA obtained was purified by gel filtration.

### (2) Preparation of DNA chip

A DNA chip was prepared by discharging the bromine labeled single strand DNA on a slide glass as a substrate on which polylysine was coated (Sigma Aldrich Japan Co.) in place of the EMCS treated substrate using the bubble jet method by the same method as in Example 4. After allowing the substrate on which the DNA solution was discharged to stand still in a moisturizing vessel for 2 hours, it was washed with pure water followed by washing with pure water. Then, the substrate was dried by blowing nitrogen gas and, after drying at 100°C for 1 hour by heating, the substrate was stored in a vacuum desiccator before used for analysis by TOF-AIMS.

### (3) Imaging and analysis by TOF-SIMS

The DNA chip in (2) was imaged and analyzed by TOF-SIMS under the same condition as in Example 4.

Only the numerical data obtained are shown in Table 4.

**Table 4**

| Ionic species | Counts |
|---|---|
| ⁷⁹Br⁻ ion | 2652 |
| ⁸¹Br⁻ ion | 2420 |
| Total | 5072 |

Table 4 shows that the DNA chip comprising the bromine labeled nucleic acid probe derived from the genome can be quantitatively determined by TOF-SIMS.

By approximately the same method as in this example, labeling with the halogen atom and imaging and quantitative analysis by TOF-SIMS are also possible with respect to the cDNA derived from mRNA. (Example 7) Preparation of Nucleic Acid Probe Array

Cleaning (1) and surface treatment (2) of the substrate were performed by the same procedure as in Example 1.

### (3) Synthesis of probe DNA

A single strand nucleic acid having the following sequence No: 9 (a nucleic acid having five molecules of 5-fluoro-3'-deoxyuridine U(F) linked at the 3'-end of 35-mer of dT) with a base length of 40 was synthesized by requesting to the DNA synthesis company (BEX). A thiol base (SH) was introduced at the 5'-terminal of sequence No: 9 single strand DNA in the synthesis step using a thiol modifier (Glen Research). After the synthesis of DNA, the DNA was deprotected and recovered by usual method, and purified by HPLC. A series of steps from the synthesis to purification were requested to the synthesis company.

U(F) was introduced at the 3'-terminal using phosphoroamidite (Glen Research) having the structure shown below.

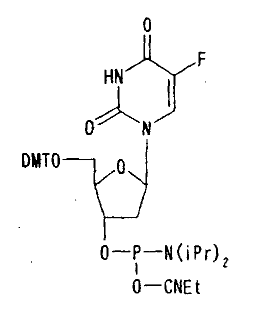

The 5-position as the fluorine substitution site introduced in place of thymine is known not to affect hybridization, and the same hybridization as in 40-mer of dT is possible.

[Sequence No: 9]
5′ HS-(CH2)6-O-PO2-O-TTTTTTTTTT TTTTTTTTTT
TTTTTTTTTT TTTTU(F)U(F)U(F)U(F)U(F)T 3′

### (4) Discharge of DNA and binding to substrate by thermal jet printer

The single strand DNA of sequence No: 9 described in (3) was dissolved in a solution containing 7.5% by weight of glycerin, 7.5% by weight of urea, 7.5% by weight of thiodiglycol and 1% by weight of acetylene alcohol (trade name: Acetylenol EH produced by Kawaken Fine Chemical Co.) in each final concentration of 10 µM, 5 µM, 2.5 µM, 1.25 µM and 0.625 µM.

Using the discharge drawing machine used in Example 1, 100 µL each of the DNA solutions was filled in the reconstructed tank, and the EMCS treated substrate was mounted on the discharge drawing machine, and the single strand DNA solutions were spotted on the surface of the EMSC treated substrate. The discharge volume during spotting was 4 picoliter/drop, and the solution was discharged at 200 dpi, or 127 µm pitch, in a 10 mm × 10 mm range of spotting at the center of the substrate. The diameter of the dot spotted under the condition above was about 50 µm.

After completing to spot, the substrate was allowed to stand still in a moisturizing chamber for 30 minutes to allow the maleimide group on the surface of the substrate to react with the sulphanyl group (-SH) at the 5'-terminal of the nucleic acid probe to immobilize the DNA probe. Subsequently, each substrate was washed with pure water, and was stored in a 50 mM phosphate buffer solution (pH = 7, solution A above) containing 1M NaCl. Immediately before analysis by TOF-SIMS, the DNA bonded substrate (DNA chip) was dried by blowing nitrogen gas, and was further dried in a vacuum desiccator.

### (Example 8) Hybridization Reaction, and Imaging and Quantitative Analysis by TOF-SIMS

### (1) Synthesis of model target nucleic acid

A model target nucleic acid (sequence No: 10 below; 40-mer of dA) comprising, at the 5'-terminal side, five adenine bases modified with the bromine atoms was synthesized by requesting to the synthesis company (BEX). The five bromine modified bases at the 5'-terminal side were introduced using 8-bromo-3'-deoxyadenosine phosphoroamidite (Glen Research) having the structure shown below in the synthesis step using an automatic synthesizer. The nucleic acid was deprotected and recovered by the usual method, and was purified by HPLC. A series of steps from the synthesis to purification were requested to the synthesis company. A(Br) in the sequence denotes deoxyadenosine modified with bromine. The 8-position of adenine as a modification site is known not to inhibit hybridization.

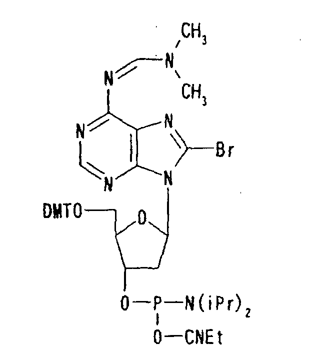

[Sequence No: 10]
5′ A(Br)A(Br)A(Br)A(Br)A(Br)AAAAA AAAAAAAAAA
AAAAAAAAAA AAAAAAAAAA 3′

### (2) Blocking and hybridization

The DNA chip prepared in Example 7 was soaked in solution A containing 2% bovine thymus albumin (BSA) at room temperature for 3 hours. After blocking the surface of the chip (for non-specific adsorption of nucleic acids), the chip was rinsed with solution A. The chip was soaked in solution A in which the model target nucleic acid of sequence No: 10 was dissolved in a concentration of 50 nM to effect hybridization at 45°C for 15 hours. Then, after rinsing the chip with pure water (at room temperature), it was dried by blowing nitrogen gas, and was stored in a vacuum desiccator before use for analysis by TOF-SIMS.

### (3) Analysis by TOF-SIMS

The DNA chip after hybridization was imaged and analyzed using the TOF-SIMS IV apparatus manufactured by ION TOF Co.

The apparatus and conditions used for the measurement are summarized below:
(Primary ion)
   primary ion: 25 kV, Ga⁺, random scan mode
   primary ion pulse frequency: 2.5 kHz (400 µsec/shot)
   primary ion pulse width: 1 ns
   primary ion diameter: 5 µm
(Secondary ion) Imaging by reconstruction of the primary ion irradiation pattern
   secondary ion detection mode: negative
   measuring region: 300 µm × 300 µm
   pixel number of secondary ion image: 128 × 128 number of integration: 256

### (4) Results

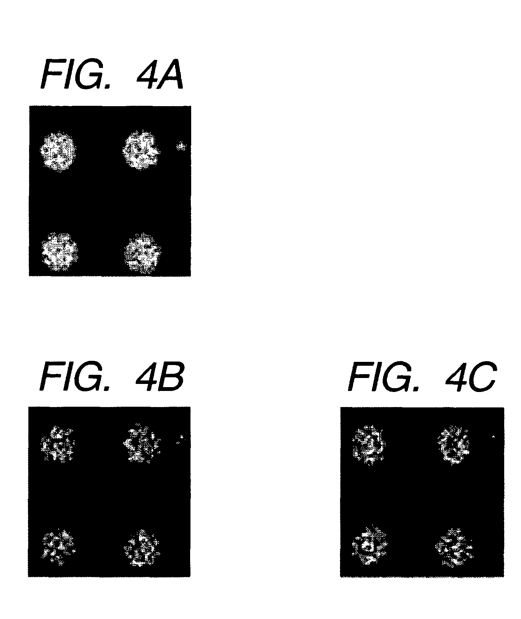

The DNA chip, prepared from the DNA solution with a nucleic acid probe concentration of 5 µm used for hybridization in (2), was analyzed by the TOF-SIMS IV apparatus under the condition above. The fluorine ion derived from the probe DNA, and the bromine ions derived from the target DNA were subjected to two dimensional imaging based on the data obtained. The results are shown in Figs. 4A to 4C. Fig. 4A shows an imaging picture of the fluorine ion (F⁻), and Figs. 4B and 4C show imaging pictures of the ⁷⁹Br⁻ ion and ⁸¹Br⁻ ion, respectively.

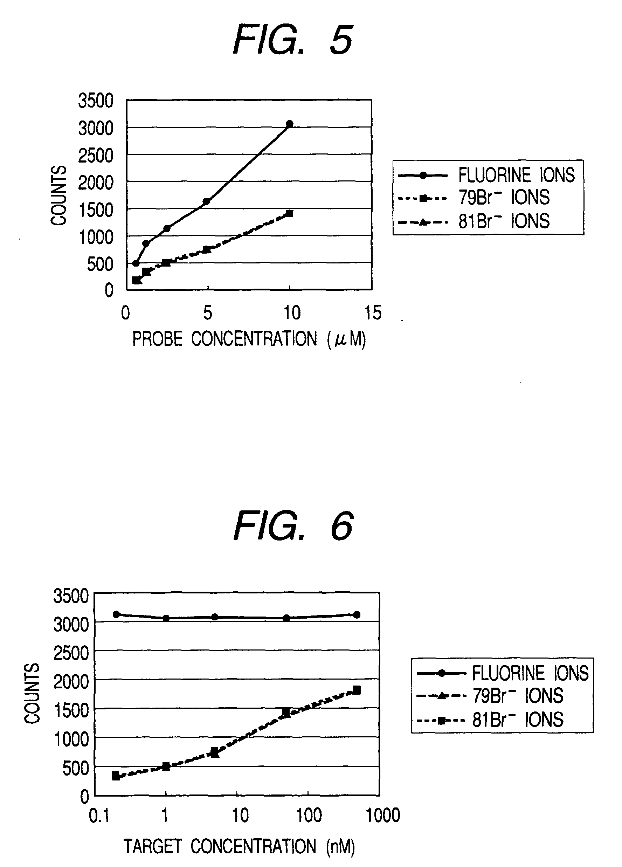

Five kind of DNA chips prepared from the DNA solutions having different nucleic acid probe concentrations, respectively, described in Example 7 were hybridized. Fig. 5 shows the plots of the counts of the fluorine ion, ⁷⁹Br⁻ ion and ⁸¹Br⁻ ion, respectively, detected by TOF-SIMS from one spot on each chip against the concentration of the nucleic acid probe used.

Figs. 4A to 4C show that the nucleic acid probe on the nucleic acid chip, and the target nucleic acid forming a hybrid with the nucleic acid probe can be simultaneously and independently imaged after forming the hybrid by taking advantage of marker atoms labeled in the nucleic acid chip. The hybrid itself containing both of the nucleic acid probe and the target nucleic acid may be imaged by integration of the image, although this method is not shown in the drawing. In addition, fragments derived from the phosphate backbone of each nucleic acid, and fragments derived from the nucleic acid base may be also observed.

Fig. 5 shows that the amount of the immobilized probe nucleic acid and the target nucleic acid on each spot can be simultaneously and independently quantified.

### (Example 9) Imaging and Quantitative Analysis of Hybrids from Samples Having Different Target Nucleic Acid Concentrations

The bromine labeled target DNA described in Example 8 was hybridized with the DNA chip prepared from the DNA solution with the probe nucleic acid concentration of 10 µM as described in Example 7 under the conditions of the target nucleic acid concentrations of 500 nM, 50 nM, 5 nM, 1nM and 0.2 nM, respectively. The DNA chips after hybridization were imaged and quantitatively analyzed by TOF-SIMS.

The counts of the fluorine ion, ⁷⁹Br⁻ ion and ⁸¹Br⁻ ion detected by TOF-SIMS were plotted against the target nucleic acid concentrations based on the quantitative analysis results. The result of plotting is shown in Fig. 6. Fig. 6 shows that the probe concentrations (the counts of the fluorine ion) on the DNA chips are approximately constant among the substrates, in contrast, according to the target nucleic acid concentrations used while the changes of the quantity of the hybrid can be quantified from the counts of the bromine ion.

### (Example 10) Imaging and Quantitative Analysis after Hybridization against Target DNA Derived from Genome: Model System

### (1) Preparation of the nucleic acid chip for detecting the target DNA derived from the genome

The fluorine labeled oligonucleotide chip was prepared according to the method for detecting the fluorescence labeled DNA described in Nature Biotechnology Vol. 18, 438, 2000 cited in Example 3. A model target logic nucleotide labeled with bromine was also synthesized, and the oligonucleotide chip and the model target oligonucleotide were hybridized.

The detailed procedure thereof will be described below:

### (1) Synthesis of fluorine labeled DNA probe and preparation of DNA chip

The DNA of sequence No: 11 was prepared as a fluorine labeled nucleic acid probe by the same method as described in Example 7. The DNA, into which a sulfanyl group is introduced at the 5'-terminal for immobilizing to the substrate and to which five fluorine labeled deoxyuridine molecules were bonded in place of the thymine molecules, comprises a base sequence complementary to a part of the base sequence contained in exon 7 of HSC4 (the part containing codon No. 248). A DNA chip was prepared using the DNA of sequence No: 11 by the same procedure as in Example 7. The concentration of the probe DNA in the solution used for preparing the chip was 10 µM.

[Sequence No: 11]
5′ HS-(CH2)6-O-PO2-O-
GAU(F)GGGCCU(F)CCGGU(F)U(F)CAU(F)G 3′

### (2) Synthesis and hybridization of bromine labeled model target DNA derived from genome

A labeled model DNA of sequence No: 12 was synthesized by the same method as described in Example 8 as the bromine labeled model target DNA. The labeled model DNA had a sequence complementary to the base sequence of the DNA of sequence No: 11, and in total of five deoxyadenosine labeled with bromine were bonded to the DNA in place of the adenosine. The bromine labeled model target DNA was hybridized with the DNA chip described in (1) above under the same condition as in Example 8 (the target DNA concentration of 50 nm), and the chip was analyzed by TOF-SIMS after hybridization.

[Sequence No: 12]
3' CTA(Br)CCCGGA(Br)GGCCA(Br)A(Br)GTA(Br)C 5'

### (3) Imaging and quantitative analysis by TOF-SIMS

The chip was subjected to blocking and hybridization under the same condition as in Example 8, and the DNA chip after hybridization was imaged and quantitatively analyzed by TOF-SIMS.

The data of counts of the fluorine ion, ⁷⁹Br⁻ ion and ⁸¹Br⁻ ion, respectively, obtained from the results of the quantitative analysis are shown in Table 5.

**Table 5**

| | |
|---|---|
| F⁻ ion | 3150 |
| ⁷⁹Br⁻ ion | 1450 |
| ⁸¹Br⁻ ion | 1432 |

Table 5 shows approximately the same results as the results of analysis under the same hybridization and analysis conditions in Example 8 and Example 9. It was confirmed from the analytical method of the invention that the probe DNA and the target DNA can be independently analyzed by independently labeling with the halogen atoms with respect to a set of the base sequences in which four kinds of the bases are mixed together, as in the probe DNA and the target DNA of practical uses.

### (Example 11) Imaging and Quantitative Analysis of Target DNA Derived from Genome after Hybridization

Imaging and quantitative analysis of the practical target DNA derived from genome will be described in this example using the DNA chip for analyzing the target DNA derived from the genome prepared in Example 10.

### (1) Preparation of bromine labeled target DNA derived from genome

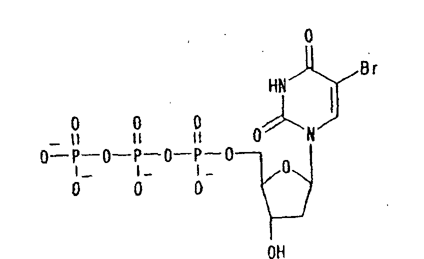

[Sequence No: 4]
E7S: 5'-ACTGGCCTCATCTTGGGCCT-3' (exon 7, sense)

[Sequence No: 5]
E7A: 5′-TGTGCAGGGTGGCAAGTGGC-3′ (exon 7, anti-
sense)

5-bromo-2'-deoxyuridine triphosphate (Br-dUTP)

The exon 7 part was synthesized from the HSC4 genome by a PCR reaction using PCR primers of sequence Nos: 4 and 5 (requested to BEX). A PCR mixture (50 µL) containing 20 ng of a genome DNA and 0.4 µM each of a sense primer or an anti-sense primer was amplified by PCR by repeating 40 cycles of 94°C (30 seconds) and 60°C (45 second) reactions. The amplification product obtained was designed to have a length of 171 nucleotides.

Then, the 0.2 µM of a sense primer (sequence NO: 12) and 10 µM of 5-bromo-2'-deoxyuridine triphosphate (Sigma Aldrich Japan Co.) as a kind of the bromine labeled nucleotide having the structure shown above were used for ssPCR (single strand PCR) by adding the other three kinds of nucleic acid bases. The PCR cycles were 25 cycles of 96°C (30 seconds), 50°C (30 seconds) and 60°C (4 minutes). The bromine labeled single strand DNA was purified by gel filtration. All the thymine bases were replaced with bromine labeled uridine in the chain elongated from the sense primer.

### (2) Blocking and hybridization

The DNA chip prepared in Example 10 was rinsed with pure water after blocking by the same method as in Example 7, and used for the following hybridization procedure.

The DNA chip after blocking was soaked six times in the SSPE solution (0.9M NaCl, 60 mM NaH₂PO₄, 6 mM EDTA) containing 20% of formamide. The solution contained the bromine labeled single strand DNA derived from the genome dissolved in a concentration of 10 nM. The solution was heated at 80°C for 10 minutes followed by hybridization at 45°C for 15 hours. The DNA chip was washed with the SSPE solution twice at 55°C thereafter using the SSPE solution followed by gently rinsing with pure water (at room temperature). After drying the DNA chip after hybridization by blowing nitrogen gas, it was stored in a vacuum desiccator before use for analysis by TOF-SIMS.

### (3) Imaging and quantitative analysis by TOF-SIMS

The DNA chip after hybridization was imaged and quantitatively analyzed by TOF-SIMS under the same condition as in Example 8.

Table 6 shows the data of counts of the fluorine ion, ⁷⁹Br⁻ ion and ⁸¹Br⁻ ion obtained from the quantitative analysis data.

**Table 6**

| | |
|---|---|
| F⁻ ion | 1267 |
| ⁷⁹Br⁻ ion | 502 |
| ⁸¹Br⁻ ion | 555 |

Table 6 shows that both the probe DNA and target DNA immobilized on the DNA chip can be independently detected and analyzed by TOF-SIMS, by applying the analysis method of the invention after hybridization of the bromine labeled target DNA derived from the genome on the DNA comprising the fluorine labeled probe.

The cDNA derived from the mRNA can be also independently imaged and analyzed by TOF-SIMS of the nucleic acid probe and target DNA after hybridization by applying approximately the same analysis method as in this example, by preparing the nucleic acid probe labeled with the halogen atoms and a chip thereof, and by PCR amplification of the target DNA labeled with a different halogen atom.

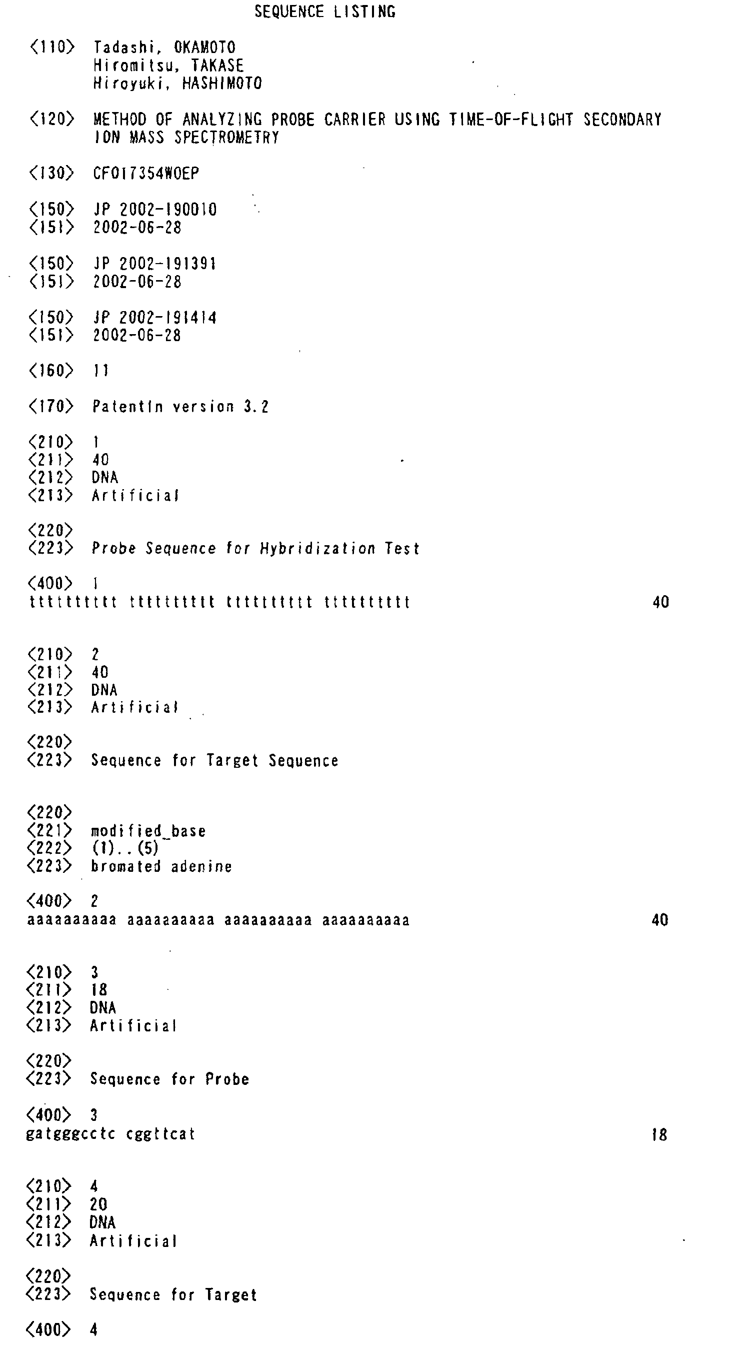

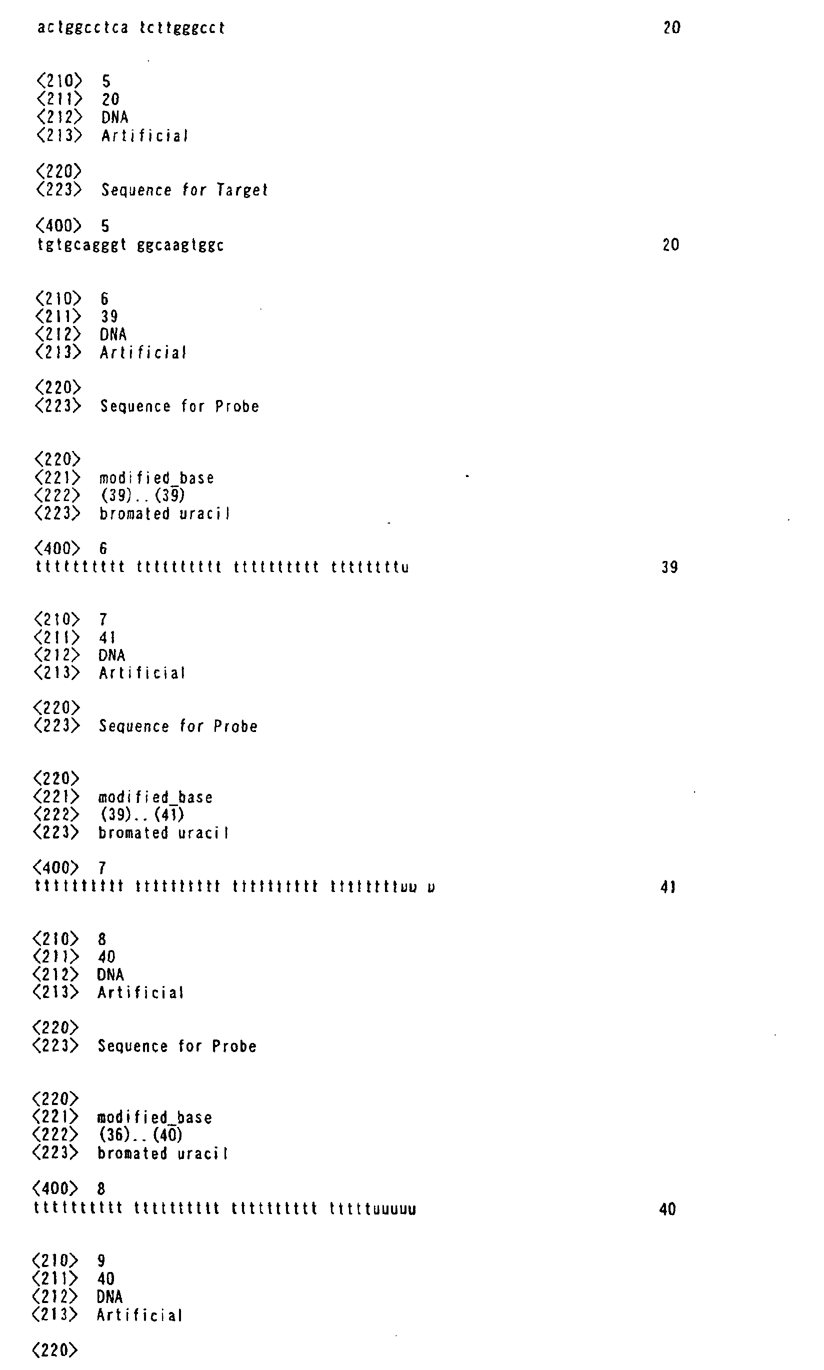

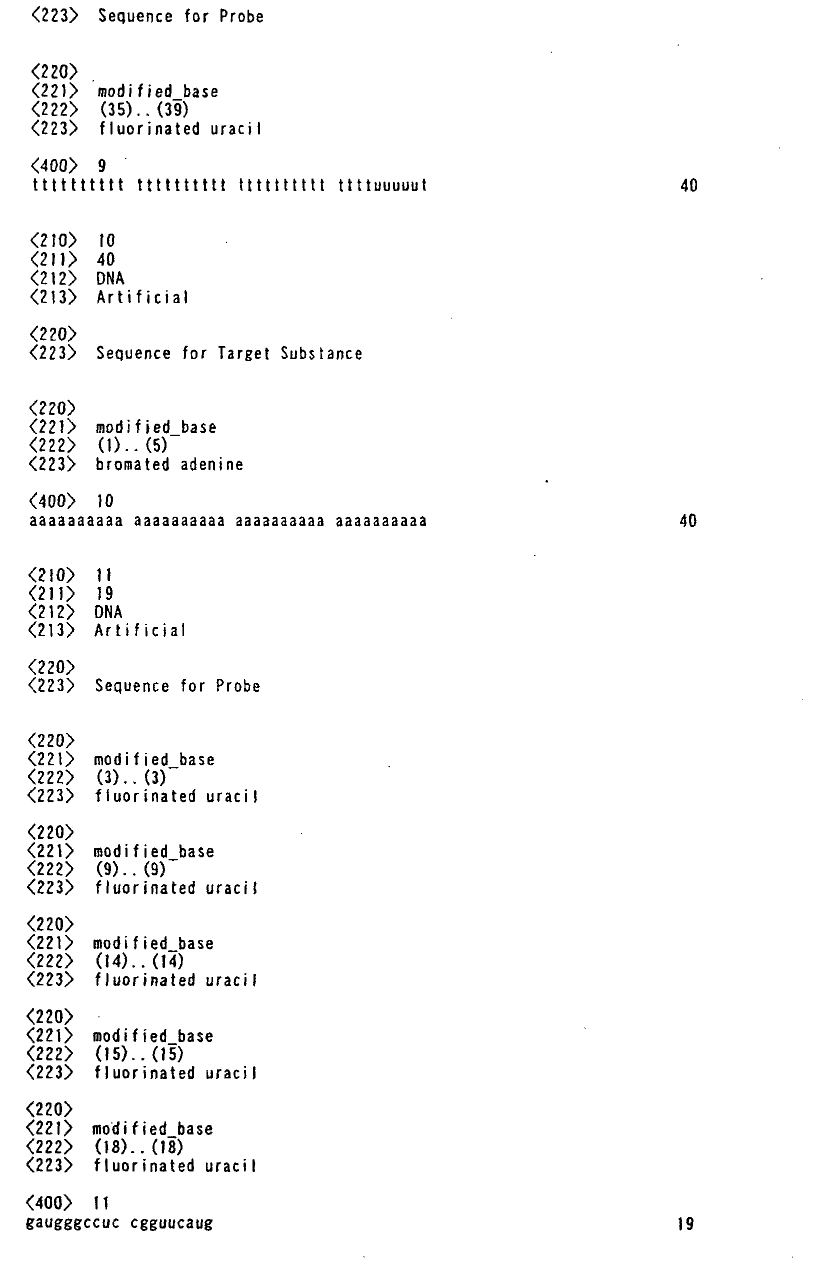

## Claims

1. A method of detecting at least one of a probe and a target substance capable of specifically binding to the probe disposed on a substrate, the method comprising the steps of:
preparing a substrate having at least one of a probe and a target substance specifically bonded to the probe disposed on a surface thereof; and
measuring the surface of the substrate by the Time-of-Flight Secondary Ion Mass Spectrometry,
wherein at least one of the probe and the target substance is labeled with a marker substance capable of forming a fragment ion that is not formed by fragmentation of the at least one of the probe and the target substance.

2. A method comprising reacting a sample with a probe carrier having a number of probe-immobilized regions disposed independently in a matrix pattern on a carrier and analyzing an analysis sample (carrier) obtained by the reaction, wherein a target substance in the sample capable of specifically binding to the probe is labeled with a halogen atom and formation/unformation of a complex obtained by the reaction between the probe and the target substance is detected by measuring the halogen atom by the Time-of-Flight Secondary Ion Mass Spectrometry.

3. A method of analyzing a probe carrier having a number of probe-immobilized regions disposed in a matrix pattern on a carrier by the Time-of-Flight Secondary Ion Mass Spectrometry, which comprises labeling the probes with halogen atoms and detecting fragment ions of the halogen atoms to analyze the state of the probe.

4. The analysis method according to Claim 2 or 3, wherein at least one of the probe and the target substance is a nucleic acid.

5. A method of analyzing a nucleic acid chip comprising a plurality of nucleic acid probes disposed in a matrix pattern on a substrate, the method comprising the steps of:
hybridizing the nucleic acid probes with a target nucleic acid in a sample to form a hybrid; and
simultaneously analyzing the nucleic acid probes and the target nucleic acid in the state of the hybrid,
wherein the nucleic acid probes and the target nucleic acid are labeled with marker substances of different prescribed numbers and then analyzing the individual marker substances by the Time-of-Flight Secondary Ion Mass Spectrometry, thereby analyzing the labeled nucleic acid probe and the labeled target nucleic acid.

6. The method according to Claim 5, which comprises selecting and using, as the marker substances, substances capable of generating secondary ions that are distinctly distinguishable from secondary ions derived from a substance constituting the nucleic acid probe and a substance constituting the target nucleic acid.

7. The analysis method according to any one of Claims 1 to 6, wherein the analysis by the Time-of-Flight Secondary Ion Mass Spectroscopy is a quantitative analysis.

8. The analysis method according to Claim 6,
wherein the marker substances comprise halogen atoms, and the nucleic acid probes and the target nucleic acid are labeled with different halogen atoms of prescribed numbers.

9. The analysis method according to any one of Claims 1 to 8, comprising sequentially pulse-irradiating entirely an analysis region of the carrier or the nucleic acid chip with primary ions as a spot having a relatively small area than the area of the analysis region; and subjecting secondary ions generated by the pulse-irradiation to time-of-flight mass spectroscopy for every pulse irradiation to effect imaging.

10. The analysis method according to Claim 9,
wherein the pulse-irradiation with the primary ions is carried out based on a non-continuous pattern, and the results of the respective mass spectroscopic analysis obtained are reconstructed based on the non-continuous pattern of the pulse-irradiation with the primary ions to effect imaging.

11. The analysis method according to Claim 10,
wherein the non-continuous pattern is a random pattern.

12. The analysis method according to Claim 11,
wherein the non-continuous pattern is a specifically programmed pattern.

13. The analysis method according to any one of Claims 2 to 4 and 8 to 12, wherein the halogen atom is any one of fluorine, chlorine, bromine and iodine atoms.

14. The analysis method according to Claim 8,
wherein the prescribed numbers of the halogen atoms for labeling the nucleic acid probes and the target nucleic acid are each within the range from 1 to the number of nucleotides constituting the nucleic acid probes and the target nucleic acid.

15. The analysis method according to Claim 14,
wherein the prescribed number of the halogen atom is 1 to 5.

16. The analysis method according to any one of Claims 4 and 8 to 15, wherein the halogen atom is bonded to at least one of the nucleotide bases of the nucleic acid probe and of the target nucleic acid.

17. The analysis method according to Claim 16,
wherein the halogen atom is bonded at a position not inhibiting the nucleic acid probe from being hybridized when hybridizing the nucleic acid probe with the target nucleic acid.

18. The analysis method according to Claim 17,
wherein the halogen atom is bonded at the 5-position of a pyrimidine base or the 8-position of a purine base.

19. The analysis method according to Claim 18,
wherein the at least one of the nucleic acid probe and the target nucleic acid is a synthetic DNA, and the halogen atom is introduced into the synthetic DNA using 2'-deoxyribonucleoside-3'-phosphoroamidite as a synthetic unit having the halogen atom bonded thereto upon synthesis of the synthetic DNA using an automatic DNA synthesizer.

20. The analysis method according to Claim 18,
wherein the synthetic unit having the halogen atom bonded thereto is represented by one of the following structural formulas: 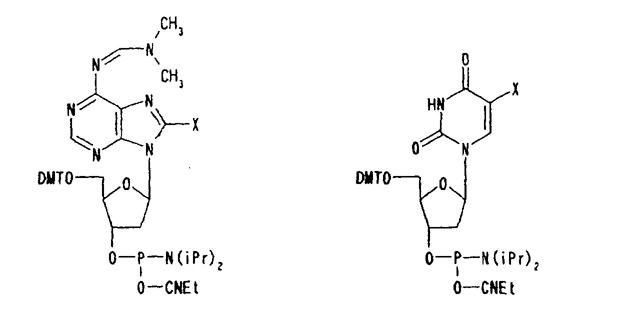 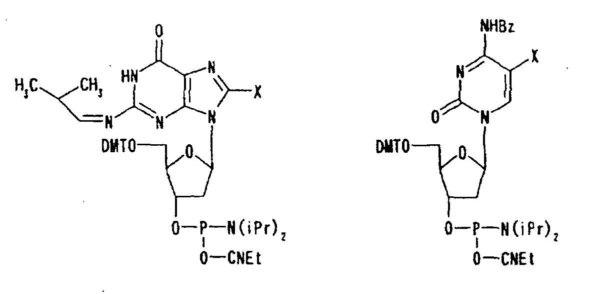 wherein X represents the halogen atom, DMTO represents a dimethoxytrityl group, iPr represents an isopropyl group, and CNEt represents a 2-cyanoethyl group.

21. The analysis method according to Claim 18,
wherein the at least one of the nucleic acid probe and the target nucleic acid is a synthetic RNA, and the halogen atom is introduced into the synthetic RNA using ribonucleoside-3'-phosphoroamidite as a synthetic unit having the halogen atom bonded thereto upon synthesis of the synthetic RNA using an automatic RNA synthesizer.

22. The analysis method according to Claim 18,
wherein the at least one of the nucleic acid probe and the target nucleic acid is a synthetic PNA, and the halogen atom is introduced into the synthetic PNA using a nucleic acid base-bonded peptide analogue as a synthetic unit having the halogen atom bonded thereto upon synthesis of the synthetic PNA using an automatic PNA synthesizer.

23. The analysis method according to Claim 18,
wherein the at least one of the nucleic acid probe and the target nucleic acid is a cDNA, and the halogen atom is introduced into the cDNA using 2'-deoxyribonucleoside-5'-triphosphate having the halogen atom bonded thereto upon synthetic elongation of the cDNA using reverse transcriptase.

24. The analysis method according to Claim 14,
wherein the at least one of the nucleic acid probe and the target nucleic acid is a DNA derived from a genome DNA, and the halogen atom is introduced into the DNA using 2'-deoxyribonucleoside-5'-triphosphate having the halogen atom bonded thereto upon synthetic elongation of the DNA with DNA polymerase.

25. The analysis method according to Claim 18,
wherein the at least one of the nucleic acid probe and the target nucleic acid is a cRNA, and the halogen atom is introduced into the cRNA using ribonucleoside-5'-triphosphate having the halogen atom bonded thereto upon synthetic elongation of the cRNA with RNA polymerase.

26. The analysis method according to Claim 14,
wherein the at least one of the nucleic acid probe and the target nucleic acid is a DNA derived from cDNA, and the halogen atom is introduced into the DNA using 2'-deoxyribonucleoside-5'-triphosphate having the halogen atom bonded thereto upon synthetic elongation of the DNA with DNA polymerase.

27. The method according to Claim 9, wherein at least one of the marker substances for labeling the nucleic acid probe and the target nucleic acid, respectively, is a metal or a metallic compound.

28. The method according to Claim 22, wherein the metal element of the metal or metallic compound is selected from the group consisting of Au, Ag, Cu, Ni, Co, Cr, Al, Ta, Pt, Pd, Zn, Sn, Ru and Rh.

29. The method according to Claim 22, wherein the metallic compound is an organic metal complex.

30. The method according to Claim 29, wherein the organic metal complex is a comples containing a metal element selected from the group consisting of Au, Ag, Cu, Ni, Co, Cr, Al, Ta, Pt, Pd, Zn, Sn, Ru and Rh.
